(19) Europäisches Patentamt
European Patent Office
Office européen des brevets

(11) **EP 4 170 221 B1**

(12) **EUROPÄISCHE PATENTSCHRIFT**

(45) Veröffentlichungstag und Bekanntmachung des Hinweises auf die Patenterteilung:
**10.07.2024   Patentblatt 2024/28**

(21) Anmeldenummer: **21000296.0**

(22) Anmeldetag: **25.10.2021**

(51) Internationale Patentklassifikation (IPC):
**F16M 11/24** *(2006.01)*

(52) Gemeinsame Patentklassifikation (CPC):
**F16M 11/046; A47B 81/064; A61L 2/10; F16M 11/18;** A61L 2202/11; A61L 2202/14; F16P 1/06

(54) **MONITORSYSTEM MIT OPTISCHER STRAHLUNGSVORRICHTUNG**

MONITOR SYSTEM WITH OPTICAL RADIATION DEVICE

SYSTÈME D'ÉCRAN POURVU DE DISPOSITIF OPTIQUE DE RAYONNEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**26.04.2023   Patentblatt 2023/17**

(73) Patentinhaber: **Element One Multimedia GmbH 76456 Kuppenheim (DE)**

(72) Erfinder:
• **Küchel, Ralf 76467 Bietigheim (DE)**

• **Nees, Thorsten 76287 Rheinstetten (DE)**

(74) Vertreter: **Thämer, Wolfgang Zürn & Thämer Patentanwälte Adalbert-Stifter-Straße 12 76275 Ettlingen (DE)**

(56) Entgegenhaltungen:
**CN-A- 109 171 201     CN-A- 113 236 942**

**Beschreibung**

Die Erfindung betrifft ein Monitorsystem mit einem, einen Einführschacht aufweisenden Gehäuse und mit einer, einen Monitor aufweisenden Anzeigevorrichtung, wobei die Anzeigevorrichtung zwischen einer Betriebsposition, in der sie vollständig aus dem Einführschacht des Gehäuses ausgefahren ist und einer Lagerposition, in der sie vollständig im Einführschacht innerhalb des Gehäuses angeordnet ist, in einer Einfahrhubrichtung und zurück mittels einer Hebevorrichtung verfahrbar ist.

Aus der EP 3 604 889 A1 ist ein derartiges Monitorsystem bekannt. Die Anzeigevorrichtung ist in vertikaler Richtung von einer ersten Betriebsposition in eine Lagerposition und zurück verfahrbar.

Aus der CN 109 171 201 A ist ein Monitorsystem mit einem klappbaren Monitor, mit einer Reinigungsbürste und mit einer mittels eines handbetätigten Schalters einschaltbaren Ultraviolettlampe zur Desinfektion bekannt.

Der vorliegenden Erfindung liegt die Problemstellung zugrunde, die Anwendungssicherheit einer derartigen Vorrichtung zu erhöhen.

Diese Problemstellung wird mit den Merkmalen des Hauptanspruches gelöst. Dazu ist im Einführschacht mindestens eine optische Strahlungsvorrichtung angeordnet, deren Wellenlänge der optischen Strahlung im Bereich von 240 Nanometer bis 280 Nanometer einschließlich liegt. In einer Längsrichtung des Monitorsystems entspricht die Nutzlänge der optischen Strahlungsvorrichtung mindestens der Länge der Anzeigevorrichtung. Außerdem ist zwischen der optischen Strahlungsvorrichtung und einer Einfahröffnung des Einführschachts eine flexible, an die Anzeigevorrichtung anlegbare Sichtschutzblende angeordnet, deren Länge mindestens der Länge der Anzeigevorrichtung entspricht. Im Gehäuse ist ein Gehäuseschutzschalter angeordnet, der die optische Strahlungsvorrichtung beim Öffnen des Gehäuses abschaltet.

Die Anzeigevorrichtung wird mittels der Hebevorrichtung von der Betriebsposition in Richtung der Lagerungsposition und zurück verfahren. Die Sichtschutzblende legt sich an die Anzeigevorrichtung an. Beim Hineinfahren und/oder beim Herausfahren der Anzeigevorrichtung wird mittels eines bistabilen Sensorsystems die optische Strahlungsquelle zunächst eingeschaltet. Nachdem die Anzeigevorrichtung bei eingeschalteter optischer Strahlungsvorrichtung einen vorgegebenen Hub zurückgelegt hat, wird die optische Strahlungsvorrichtung wieder abgeschaltet. Der vorgegebene Hub ergibt sich aus der Höhe der Anzeigevorrichtung in der Einfahrrichtung, dem Abstand der optischen Strahlungsquelle zur Anzeigevorrichtung und dem Abstrahlwinkel der optischen Strahlungsquelle.

Während des Einsatzes des Monitorsystems, z.B. in einem Konferenzraum, steht die Anzeigevorrichtung in einer ausgefahrenen Betriebsposition. Beispielsweise kann der Bediener den berührungsempfindlichen Monitor der Anzeigevorrichtung bedienen. Zum Abschluss des Einsatzes des Monitorsystems wird die einzelne Anzeigevorrichtung von der Betriebsposition in ein Gehäuse in Richtung einer Lagerposition eingefahren. Sobald die optische Strahlungsvorrichtung eingeschaltet ist, desinfiziert deren Strahlung zumindest die dem Benutzer zugewandte Seite der Anzeigevorrichtung. Anschließend wird die optische Strahlungsvorrichtung abgeschaltet und die Anzeigevorrichtung z.B. in die Lagerposition verfahren. Zusätzlich oder alternativ ist ein Einschalten der optischen Strahlungsvorrichtung beim Herausfahren der Anzeigevorrichtung aus der Lagerposition in die Betriebsposition denkbar.

Weitere Einzelheiten der Erfindung ergeben sich aus den Unteransprüchen und der nachfolgenden Beschreibung schematisch dargestellter Ausführungsformen.

Figur 1:     Monitorsystem;
Figur 2:     Querschnitt des Monitorsystems aus Figur 1;
Figur 3:     Vereinfachte Detaildarstellung aus Figur 2;
Figur 4:     Optische Strahlungsvorrichtung;
Figur 5:     Detail eines Monitorsystems mit einem Deviationswinkel der optischen Strahlungsvorrichtung von 30 Grad.

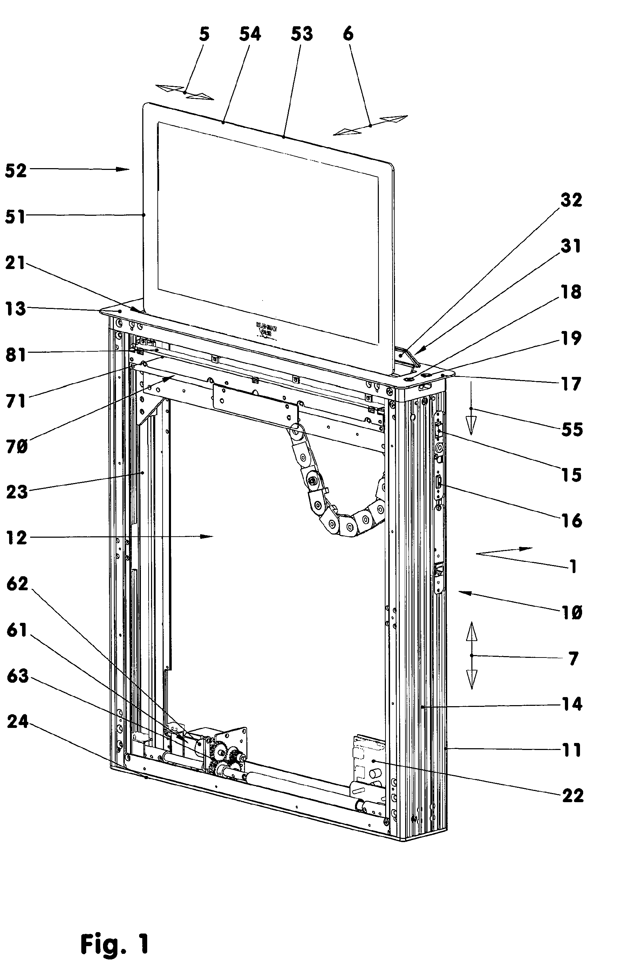

Die Figuren 1 bis 4 zeigen ein Monitorsystem (10) mit einem Gehäuse (11) und mit einer Anzeigevorrichtung (51). Derartige Monitorsysteme (10) werden beispielsweise in Möbelstücken, z.B. Tischen, angeordnet. Hierbei liegt ein am Gehäuse (11) angeordneter Auflageflansch (13) z.B. im Tisch bündig mit der Tischplatte. Beispielsweise liegt er in einer Einfräsung der Tischplatte und ist dort befestigt. Ein Einführschacht (12) des Gehäuses (11) mündet in die Ebene der Tischplatte. Im Ausführungsbeispiel ist der Einführschacht (12) in vertikaler Richtung orientiert.

Die Anzeigevorrichtung (51) ist in den Figuren 1 bis 3 in einer Betriebsposition (52) dargestellt. Sie steht in diesen Darstellungen oberhalb des Einführschachtes (12). Aus dieser Betriebsposition (52) ist die Anzeigevorrichtung (51) mittels einer Hebevorrichtung (61) in eine Lagerposition verfahrbar. In dieser Lagerposition befindet sich die Anzeigevorrichtung (51) vollständig im Einführschacht (12) innerhalb des Gehäuses (11). Auch das Wiederausfahren von der Lagerposition in die Betriebsposition (52) erfolgt mittels der Hebevorrichtung (61). In der Betriebsposition (52) kann die Anzeigevorrichtung relativ zur Hebevorrichtung (61) schwenkbar ausgebildet sein.

Das Gehäuse (11) ist im Ausführungsbeispiel quaderförmig ausgebildet und trägt den obenliegenden Auflageflansch (13). Es hat - ohne den Auflageflansch (13) - eine in der Längsrichtung (5) orientierte Länge von z.B. 510 Millimeter, eine in der Querrichtung (6) orientierte Breite von beispielsweise 80 Millimeter und eine in der Höhenrichtung (7) orientierte Höhe von 600 Millimeter.

Der Auflageflansch (13) ist in der Draufsicht ein Rechteck mit abgerundeten Ecken. Seine Länge ist z.B. 4% länger als die oben genannte Länge des Gehäuses (11). Seine Breite ist beispielsweise um 25 % größer als die Breite des Gehäuses (11).

Am den vertikalen Schmalseiten (14) des Gehäuses (11) sind beispielsweise die elektrischen Anschlüsse (15), Anschlüsse (16) für Datenleitungen, etc. angeordnet. Bei eingebautem Monitorsystem (10) liegen diese Anschlüsse (15, 16) unterhalb der Tischplatte.

An der Oberseite (17) des Gehäuses (11) sind im Auflageflansch (13) Betätigungstaster (18), Betriebsanzeigen (19), etc. angeordnet. Mittels des Betätigungstasters (18) kann beispielsweise das Einfahren der Anzeigevorrichtung (51) in das Gehäuse (11) und/oder das Ausfahren der Anzeigevorrichtung (51) aus dem Gehäuse (11) eingeleitet werden. Auch eine . Fernsteuerung des Ein- und/oder Ausfahrens ist alternativ oder zusätzlich denkbar.

Im Auflageflansch (13) ist eine Einfahröffnung (21) des Einführschachts (12) als Durchbruch ausgebildet. Die Länge dieser Einfahröffnung (21) beträgt beispielsweise 88 % der obengenannten Länge des Gehäuses (11). Ihre Breite entspricht der Hälfte der Breite des Gehäuses (11). Die Einfahröffnung (21) hat im Ausführungsbeispiel einen rechteckigen Querschnitt mit abgerundeten Ecken.

Die Einfahröffnung (21) ist im Ausführungsbeispiel mittels einer Verschlussklappe (31) verschließbar. Die Einfahröffnung (21) kann auch ohne Verschlussklappe (31) ausgebildet sein. In den Darstellungen der Figuren 1 bis 3 ist die Verschlussklappe (31) im geöffneten Zustand dargestellt. Die Verschlussklappe (31) weist eine Verschlussplatte (32), zwei Verschlusshebel (33), einen Verschlussantrieb (34) und eine Verschlussüberwachung auf. Die Verschlussplatte (32) ist eine ebene Platte, deren Abmessungen geringfügig kleiner sind als die Abmessungen der Einfahröffnung (21). An den in der Längsrichtung (5) orientierten Enden der Verschlussplatte (32) ist jeweils ein Verschlusshebel (33) angeordnet. Der Verschlussantrieb (34) hat einen z.B. elektrischen Verschlussmotor. Dieser treibt beispielsweise mittels eines nachgeschalteten Getriebes die Verschlusshebel (33) an. Die Verschlussüberwachung hat z.B. zwei Schalter in der Bauform von End- oder Näherungsschaltern. Ein erster dieser Schalter schaltet bei vollständig geöffneter Verschlussklappe (31). Der zweite Schalter wird bei vollständig geschlossener Verschlussklappe (31) betätigt. Die Signale beider Schalter werden an ein im Gehäuse (11) angeordnetes Steuerungsmodul (22) zur Freigabe von Folgefunktionen weitergeleitet.

Im unteren Bereich des Gehäuses (11) ist die Hebevorrichtung (61) angeordnet. Die Hebevorrichtung (61) hat beispielsweise einen Elektromotor (62) als Antriebsmotor. Auch eine hydraulische, pneumatische, magnetische, etc. Antriebseinheit der Hebevorrichtung (61) ist denkbar. Im Ausführungsbeispiel ist als Elektromotor (61) ein Schrittmotor eingesetzt, dessen Schrittweite 1,8 Grad beträgt. Das Haltemoment dieses Elektromotors (62) beträgt beispielsweise 0,72 Newtonmeter. Der Elektromotor (62) treibt mittels eines z.B. mehrstufigen Stirnradgetriebes (63) beispielsweise eine Hubspindel, einen Fördergurt, eine Zylinder-Kolben-Einheit etc. Die Hebevorrichtung (61) hat zwei Tragpratzen (64), an denen die Anzeigevorrichtung (51) befestigt ist.

Beispielsweise im Einführschacht (12) des Gehäuses (11) ist ein Sensorsystem (40) mit z.B. zwei Schaltern (41, 42) in der Bauform von Mikroschaltern angeordnet. Für die Betätigung haben die Schalter (41, 42) beispielsweise einen pilzförmigen Kopf (43). Auch der Einsatz von Näherungsschaltern ist denkbar. Ein erster dieser Schalter (41) ist unterhalb der Verschlussklappe (31) angeordnet. Er wird beim Passieren der Anzeigevorrichtung (51) betätigt. Der zweite Schalter (42) wird bei fast vollständig eingefahrener Anzeigevorrichtung (51) betätigt. Die Signale beider Schalter (41, 42) werden an das im Gehäuse (11) angeordnete Steuerungsmodul (22) zur Freigabe von Folgefunktionen weitergeleitet. Der minimale Abstand der Schalter (41, 42) zueinander in der Einfahrhubrichtung (55), der Anzeigevorrichtung (51) entspricht der Höhe der Anzeigevorrichtung (51) in dieser Einfahrhubrichtung (55), reduziert um das Produkt aus dem Abstand (a) einer Lichtquelle (75) der optischen Strahlungsquelle (70) zu einer Anzeigeebene (59) der Anzeigevorrichtung (51) und dem Tangens des halben Öffnungswinkels ($\alpha$) der optischen Strahlungsquelle (70). Der minimal erforderliche Abstand der Schalter (41, 42) zueinander wird im Folgenden als minimaler Schaltabstand (44) bezeichnet.

Das Sensorsystem (40) kann auch als absolutes Messsystem im Einführschacht (12) oder an der Hebevorrichtung (61) ausgebildet sein. Dies kann z.B. ein absoluter Drehgeber sein, der auf der Welle des Antriebsmotors (62) sitzt. Auch der Einsatz eines relativen Messsystems, beispielsweise ausgehend von einer Hubendlage der Anzeigevorrichtung (51), ist denkbar. Das Monitorsystem (10) kann auch ohne ein Sensorsystem (40) ausgebildet sein. In diesem Fall wird die optische Strahlungsquelle (70) beispielsweise mittels des Betätigungstasters (18) oder manuell eingeschaltet. Das Abschalten der optischen Strahlungsvorrichtung (70) erfolgt dann z.B. beim Erreichen der Lagerposition oder manuell.

Der Wandungen (23) des Einführschachtes (12) sind beispielsweise mit matten Oberflächen ausgebildet. Beispielsweise können die Oberfläche mittels Fräs- oder Schleifverfahren bearbeitet sein. Gegebenenfalls können die Wandungen (23) alternativ oder zusätzlich mit einer schwarzen oder dunklen Schicht beschichtet sein.

Die Anzeigevorrichtung (51) weist im Ausführungsbeispiel einen Monitor (53) auf. Dieser ist z.B. als Flachbildschirm mit einer berührempfindlichen Sichtoberfläche ausgebildet. Damit können Eingaben am Monitor (53) durchgeführt werden. Es ist auch denkbar, die Anzeigevorrichtung (51) z.B. für Präsentationen ohne Eingabemöglichkeit auszubilden. Auch eine Ausbildung mit z.B. zwei Monitoren, mit einer Standinformation und einem Monitor, etc. ist denkbar.

Der Monitor (53) hat eine transparente Schutzscheibe (54), die sowohl den Bildschirm (56) als auch den Rahmen (57) überdeckt. Die Schutzscheibe (54) besteht im Ausführungsbeispiel aus entspiegeltem Glas und hat eine Dicke zwischen einem und drei Millimetern.

Unterhalb der Einführöffnung (21) ist im Gehäuse (11) die optische Strahlungsvorrichtung (70) angeordnet. Die optische Strahlungsvorrichtung (70) sitzt auf einem im Einführschacht (12) des Gehäuses (11) befestigten Modulträger (71). Im Ausführungsbeispiel hat die optische Strahlungsvorrichtung (70) drei z.B. identisch zueinander ausgebildete Leuchteinheiten (72 - 74). Eine erste Leuchteinheit (72) und eine zweite Leuchteinheit (73) fluchten miteinander. Sie sind an den beiden in der Längsrichtung (5) orientierten Enden des Einführschachts (12) angeordnet. Die dritte Leuchteinheit (74) liegt symmetrisch zur vertikalen Mittenquerebene des Monitorsystems (10) unterhalb der erstgenannten Leuchteinheiten (72, 73). Die Leuchteinheiten (72 - 74) können aber auch alle miteinander fluchtend angeordnet sein. Die in der Längsrichtung (5) orientierte Nutzlänge der optischen Strahlungsvorrichtung (70) ist größer oder gleich der Länge der Anzeigevorrichtung (51) in dieser Richtung. Die Nutzlänge ist hierbei die Länge der mittels der optischen Strahlungsvorrichtung (70) ausgeleuchteten Fläche in der Längsrichtung (5).

Die einzelne Leuchteinheit (72; 73; 74) hat mindestens eine Lichtquelle (75), die in einem Trägergehäuse (76) angeordnet ist. Gegebenenfalls ist der Lichtquelle (75) oder den Lichtquellen ein optisches Linsensystem zur gleichmäßigen Ausleuchtung eines Zielfelds nachgeschaltet. Die einzelne Leuchteinheit (72; 73; 74) hat z.B. eine Länge von 145 Millimeter und eine Höhe von 19 Millimetern. Die einzelne Leuchteinheit (72; 73; 74) hat im Ausführungsbeispiel eine Anschlussleistung von 1 Watt und wird mit einer Spannung von 12 Volt betrieben. Die Lichtquelle (75) strahlt ultraviolettes Licht innerhalb einem Wellenlängenbereich von 240 Nanometer bis einschließlich 280 Nanometer ab. Im Ausführungsbeispiel beträgt die Wellenlänge des abgestrahlten Lichts 275 Nanometer. Auch der Einsatz einer Lichtquelle (75) mit einer Wellenlänge von 254 Nanometer, 265 Nanometer, 280 Nanometer, etc. ist denkbar. Die einzelne Lichtquelle (75) ist z.B. als Leuchtdiode ausgebildet. Diese sitzt beispielsweise auf einer Aluminiumplatine, die zur Wärmeabfuhr eingesetzt wird.

Die Strahlungsleistung der Leuchteinheit (72; 73; 74) beträgt im Ausführungsbeispiel 10 Milliwatt. Die Leuchteinheit (72; 73; 74) hat einen Abstrahlwinkel ($\alpha$) von 120 Grad. Im Ausführungsbeispiel ist die Winkelhalbierende des Abstrahlwinkels der Leuchteinheit (72; 73; 74) normal zur Ebene der Außenseite (58) der Schutzscheibe (54) ausgerichtet. Diese Ebene wird im Folgenden als Anzeigeebene (59) der Anzeigevorrichtung (51) bezeichnet. Es ist aber auch denkbar, die Winkelhalbierende z.B. in einem Winkel bis zu 45 Grad zu dieser Anzeigeebene anzuordnen, wobei die Abstrahlrichtung dann in Richtung der Hebevorrichtung (61) orientiert ist. Der Abstand (a) der Lichtquelle (75) zur Anzeigeebene (59) der Anzeigevorrichtung (51) beträgt beispielsweise 7 % der Breite des Gehäuses (11).

Am Modulträger (71) ist im Ausführungsbeispiel eine optische Funktionsanzeige (81) des optischen Strahlungsmoduls (70) angeordnet. Diese optische Funktionsanzeige (81) ist beispielsweise eine Leuchtdiode, die z.B. blaues Licht im sichtbaren Bereich emittiert. Die optische Funktionsanzeige (81) kann auch an der Außenseite des Gehäuses (11), im Auflageflansch (13), etc. angeordnet sein.

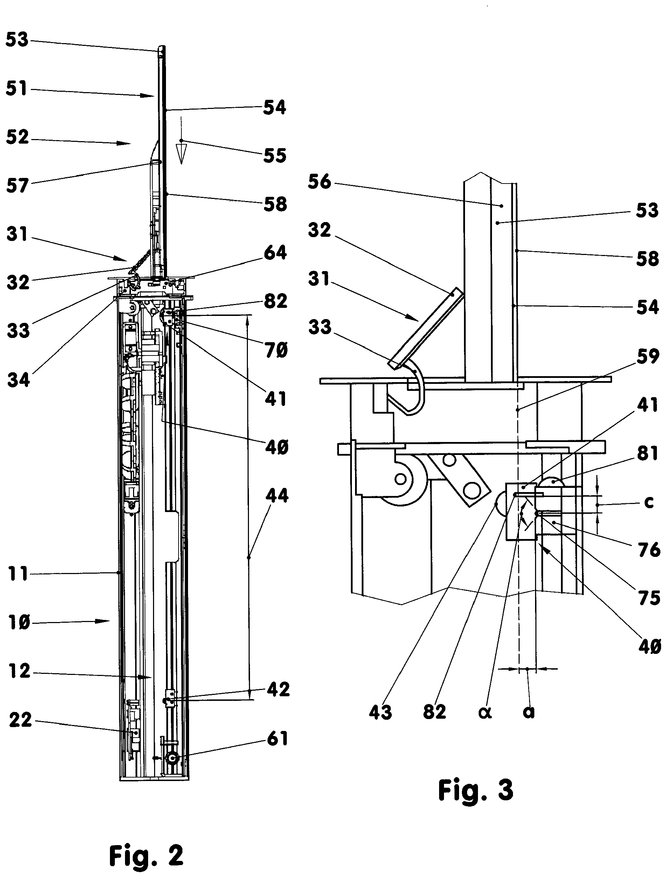

Im oberen Bereich des Modulträgers (71) ist oberhalb der optischen Strahlungsvorrichtung (70) eine Sichtschutzblende (82) befestigt. In den Darstellungen der Figuren 2 und 3 schneidet die Ebene der Sichtschutzblende (82) den ersten Schalter (41) oberhalb des Kopfes (43). Der Abstand der Ebene der Sichtschutzblende (82) zur geschlossenen Verschlussklappe (31) beträgt beispielsweise 60 % der Breite des Gehäuses (11). Im Ausführungsbeispiel ist die Sichtschutzblende (82) als Bürste ausgebildet. Ihre Länge beträgt beispielsweise 82 % der Länge des Gehäuses (11). In der Längsrichtung (5) ist die Sichtschutzblende (82) länger als die Nutzlänge der optischen Strahlungsvorrichtung (70). Die Breite der Sichtschutzblende (82) ist beispielsweise doppelt so groß wie der Abstand der Lichtquelle (75) zur Anzeigeebene (59) der Anzeigevorrichtung (51).

Der Mindestabstand (c) der Sichtschutzblende (82) zur Lichtquelle (75) in der Einfahrhubrichtung (55) der Anzeigevorrichtung (51) ergibt sich zu:

$$c > a * \tan{(\alpha/2 - \delta)}$$

mit

a: Abstand der Lichtquelle (75) zur Anzeigeebene (59) [m]

c: Abstand in der Hubrichtung zwischen der Lichtquelle (75) und der Sichtschutzblende (82) [m]

$\alpha$: Abstrahlwinkel der Lichtquelle [Grad]

$\delta$: Deviationswinkel [Grad]

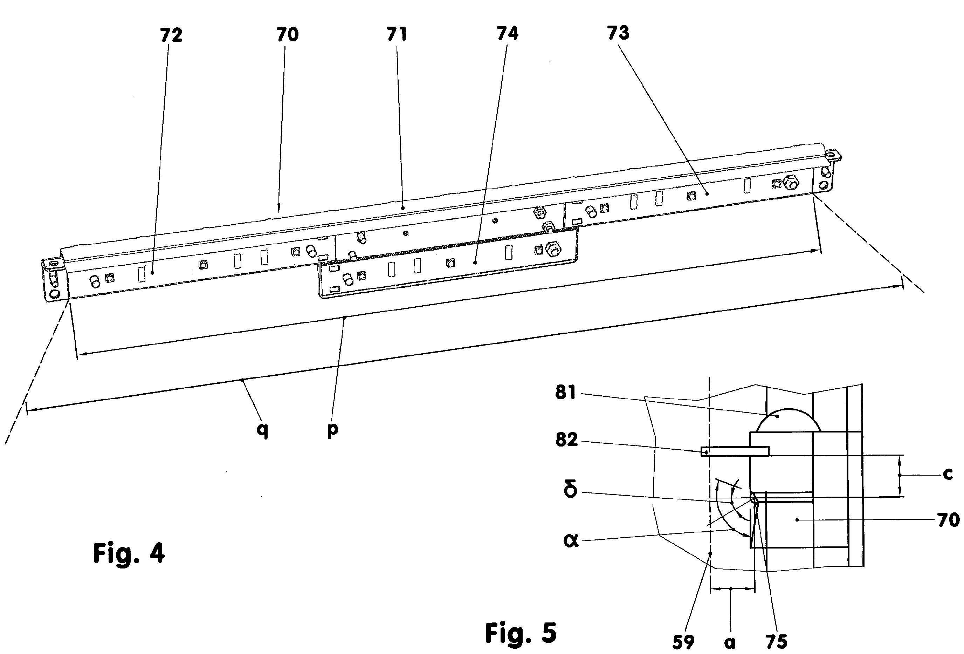

Der Mindestabstand (c) ist größer als das Produkt aus dem Abstand (a) der Lichtquelle (75) zur Anzeigeebene (59) der Anzeigevorrichtung (51) und des Tangens der Differenz des halben Abstrahlwinkels ($\alpha$) und eines Deviations-winkels ($\delta$). Der Deviationswinkel ($\delta$), vgl. Figur 5, ist hierbei der Winkel, der von einer Normalen zur Anzeigeebene (59) der Anzeigevorrichtung (51) durch die Lichtquelle (75) und der Winkelhalbierenden des Abstrahlwinkels ($\alpha$) der Licht-quelle (75) eingeschlossen wird. Im Ausführungsbeispiel der Figuren 1 - 4 beträgt der Deviationswinkel ($\delta$) Null Grad.

Um die Anzeigevorrichtung (51) aus der Betriebsposition (52) in die Lagerposition zu verfahren, wird beispielsweise der Betätigungstaster (18) im Auflageflansch (13) betätigt. Der Elektromotor (62) der Hebevorrichtung (61) wird eingeschaltet. Die Verschlussklappe (31) steht in der geöffneten Position.

Die Hebevorrichtung (61) senkt die Anzeigevorrichtung (51) ab. Im Ausführungsbeispiel beträgt die z.B. konstante Hubgeschwindigkeit der Anzeigevorrichtung (51) in der Hubrichtung 30 Millimeter pro Sekunde. Beim Absenken der Anzeigevorrichtung (51) legt sich die Sichtschutzblende (82) an die z.B. als Planfläche oder als nanostrukturierte Fläche ausgebildete Außenseite (58) der Schutzscheibe (54) an. Der Bereich des Einführschachts (12) unterhalb der Sichtschutzblende (82) ist optisch abgeschirmt gegen die Umgebung (1). Beim weiteren Verfahren der Anzeigevorrichtung (51) in Richtung der Lagerposition wird der erste Schalter (41) betätigt. Damit wird die optische Strahlungsvorrichtung (70) eingeschaltet. Auch ein manuelles Einschalten der optischen Strahlungsquelle (70) ist denkbar. Die Lichtquellen (75) der Leuchteinheiten (72, 73, 74) emittieren Licht innerhalb eines Abstrahlkegels, dessen Spitzenwinkel dem Ab-strahlwinkel ($\alpha$) entspricht. Die Sichtschutzblende (82) verhindert ein Austreten der Strahlung des optischen Strahlungsmoduls (70) in die Umgebung (1). Die optische Funktionsanzeige (81) kann für den Benutzer sichtbar sein. Beim Passieren des mittels der beschriebenen optischen Strahlungsvorrichtung (70) ausgeleuchteten Bereichs werden Keime und Kleinstorganismen, die sich auf der Anzeigevorrichtung (51) befinden, abgetötet. Um eine vorgegebene Desinfektionsrate zu erreichen, darf die Hubgeschwindigkeit (v) in der Einfahrhubrichtung (55) eine maximale Geschwindigkeit nicht überschreiten. Für die Hubgeschwindigkeit (v) gilt:

$$v < P / (D * q)$$

mit

P: Strahlungsleistung der Lichtquellen [W]
D: erforderliche Strahlungsdosis [J/m$^2$]
q: Länge der ausgeleuchteten Fläche in der Anzeigeebene (59) quer zur Hubrichtung (55), Nutzlänge von (70) [m]

Die maximal zulässige Hubgeschwindigkeit ($v_{max}$) ist kleiner als der Quotient aus der Strahlungsleistung (P) der optischen Strahlungsvorrichtung (70) und dem Produkt aus der erforderlichen Strahlungsdosis (D) und der Länge der ausgeleuchteten Fläche (q).

Die Länge der ausgeleuchteten Fläche in der Anzeigeebene (59) ist:

$$q = p + \pi/4 * a * (\tan (\alpha/2 - \delta) + \tan (\alpha/2 + \delta))$$

mit

p: Abstrahllänge des optischen Strahlungsmoduls [m]

Die Länge (q) der ausgeleuchteten Fläche in einer Richtung normal zur Einfahrhubrichtung (55) ist die Summe aus der Abstrahllänge (p) des optischen Strahlungsmoduls (70) und dem Produkt aus dem Abstand (a) der Lichtquelle (75) zur Anzeigeebene (59), einem Viertel der Kreiszahl ($\pi$) und der Summe aus dem Tangens des um den Deviations-winkel ($\delta$) verminderten halben Abstrahlwinkels ($\alpha$) und dem Tangens des um den Deviationswinkel ($\delta$) erhöhten halben Abstrahlwinkels ($\alpha$).

Im Ausführungsbeispiel entspricht die gewählte Hubgeschwindigkeit (v) einem Drittel der zulässigen Hubge-schwindigkeit ($v_{max}$), bei der eine 99 %-ige Desinfektion erreicht wird.

Kurz vor dem Erreichen der Lagerposition betätigt die Anzeigevorrichtung (51) den zweiten Schalter (42). Die optische Strahlungsvorrichtung (70) und die optische Funktionsanzeige (81) werden abgeschaltet. Beim Einsatz eines anders aufgebauten Sensorsystems (4) wird die optische Strahlungsvorrichtung (70) abgeschaltet, sobald sie nach dem Einschalten ein aufgrund des Schaltabstands (44) ausgelöstes zweites Signal erhält. Die Hebevorrichtung (61) wird z. B. noch für wenige Sekunden weiterbetrieben, sodass die Anzeigevorrichtung (51) weiter abgesenkt wird. Dann wird der Elektromotor (62) der Hebevorrichtung (61) abgeschaltet. Die Verschlussklappe (31) wird verschlossen. In der Lagerposition steht die Anzeigevorrichtung (51) beispielsweise vollständig unterhalb der Sichtschutzblende (82).

Um die Anzeigevorrichtung (51) wieder einzusetzen, wird der Betätigungstaster (18) im Auflageflansch (13) erneut betätigt. Die Verschlussklappe (31) wird geöffnet. Anschließend wird die Anzeigevorrichtung (51) mittels der Hebevorrichtung (61) entgegen der Einfahrhubrichtung (55) von der Lagerungsposition in die Betriebsposition (52) gefördert. Diese Hubbewegung erfolgt beispielsweise mit der gleichen Hubgeschwindigkeit wie die Absenkbewegung.

Beim Anheben der Anzeigevorrichtung (51) kann die optische Strahlungsvorrichtung (70) eingeschaltet werden. Beispielsweise wird sie beim Einschalten der Hebevorrichtung (61) oder zeitverzögert nach dem Einschalten des Elektromotors (62) eingeschaltet. Die Zeitverzögerung kann beispielsweise bis zu fünf Sekunden betragen. Auch kann der zweite Schalter (42) zum Einschalten der optischen Strahlungsvorrichtung (70) eingesetzt werden. Für die Auslegung der Ausfahrhubgeschwindigkeit gelten die obengenannten Zusammenhänge der Einfahrhubgeschwindigkeit.

Die Bestrahlung der Anzeigevorrichtung (51) erfolgt, wie oben beschrieben. Beim Passieren des ersten Schalters (41) werden die optische Strahlungsvorrichtung (70) und die optische Funktionsanzeige (81) wieder abgeschaltet. Die Anzeigevorrichtung (51) wird nun weiter in die Betriebsposition (52) verfahren.

Im Gehäuse (11) ist ein Gehäuseschutzschalter angeordnet. Dieser wird beim Öffnen des Gehäuses (11) für Wartungszwecke aktiviert. Bei Aktivierung dieses Gehäuseschutzschalters wird die elektrische Versorgung der optischen Strahlungsvorrichtung (70) unterbrochen.

Die optische Strahlungsvorrichtung (70) kann an ein bestehendes Monitorsystem (10) nachgerüstet werden. Hierzu wird im Einführschacht (12) des Gehäuses (11) der Modulträger (71) mit der optischen Strahlungsvorrichtung (70) und der Sichtschutzblende (82) eingesetzt und befestigt. Gegebenenfalls kann ein Sensorsystem (40) ebenfalls nachgerüstet werden. Die Steuerung des Monitorsystems (10) wird um die Funktionen der optischen Strahlungsvorrichtung (70) und des Sensorsystems (40) erweitert.

Es ist auch denkbar, im Gehäuse (11) zusätzlich zu der optischen Strahlungsvorrichtung (70) auf der Seite der Anzeigeebene (59) eine weitere optische Strahlungsvorrichtung für die Rückseite der Anzeigevorrichtung (51) anzuordnen. Diese weitere optische Strahlungsvorrichtung liegt dann beispielsweise in der gleichen, normal zur Hubrichtung (55) orientierten Ebene wie die oben beschriebene optische Strahlungsvorrichtung (70). Auch ist dann oberhalb der weiteren optischen Strahlungsvorrichtung eine Sichtschutzblende angeordnet, die z.B. in der gleichen Ebene wie die erstgenannte Sichtschutzblende (82) liegt. Das Einschalten und das Ausschalten beider optischen Strahlungsvorrichtungen (70) kann dann mittels derselben Sensorsystems (40) gesteuert werden.

Der Einführschacht (12) kann auch waagerecht oder schräg im Raum angeordnet sein. Die Einfahrhubrichtung (55) der Anzeigevorrichtung (51) ist auch in diesen Fällen von der Einfahröffnung (21) des Einführschachts (12) in Richtung des Bodens (24) des Einführschachts (12) orientiert.

Auch Kombinationen der verschiedenen Ausführungsformen sind denkbar.

Bezugszeichenliste:

| | |
|---|---|
| 1 | Umgebung |
| 5 | Längsrichtung |
| 6 | Querrichtung |
| 7 | Höhenrichtung |
| 10 | Monitorsystem |
| 11 | Gehäuse |
| 12 | Einführschacht |
| 13 | Auflageflansch |
| 14 | Schmalseiten |
| 15 | elektrische Anschlüsse |
| 16 | Datenanschlüsse |
| 17 | Oberseite von (11) |
| 18 | Betätigungstaster |
| 19 | Betriebsanzeige |
| 21 | Einfahröffnung |
| 22 | Steuerungsmodul |
| 23 | Wandung von (12) |
| 24 | Boden von (12) |
| 31 | Verschlussklappe |

| | |
|---|---|
| 32 | Verschlussplatte |
| 33 | Verschlusshebel |
| 34 | Verschlussantrieb |
| | |
| 40 | Sensorsystem |
| 41 | Schalter, erster Schalter |
| 42 | Schalter, zweiter Schalter |
| 43 | Kopf |
| 44 | Schaltabstand |
| 51 | Anzeigevorrichtung |
| 52 | Betriebsposition |
| 53 | Monitor |
| 54 | Schutzscheibe |
| 55 | Einfahrhubrichtung |
| 56 | Bildschirm |
| 57 | Rahmen |
| 58 | Außenseite |
| 59 | Anzeigeebene |
| | |
| 61 | Hebevorrichtung |
| 62 | Elektromotor |
| 63 | Stirnradgetriebe |
| 64 | Tragpratzen |
| | |
| 70 | Optische Strahlungsvorrichtung |
| 71 | Modulträger |
| 72 | Leuchteinheit, erste Leuchteinheit |
| 73 | Leuchteinheit, zweite Leuchteinheit |
| 74 | Leuchteinheit, dritte Leuchteinheit |
| 75 | Lichtquelle |
| 76 | Trägergehäuse |
| | |
| 81 | optische Funktionsanzeige |
| 82 | Sichtschutzblende |
| | |
| $\alpha$ | Öffnungswinkel von (70) |
| $\delta$ | Deviationswinkel |
| $\pi$ | Kreiszahl |
| | |
| a | Abstand zwischen (75) und (59) [m] |
| c | Abstand zwischen (75) und (82) [m] |
| p | Abstrahllänge von (70) [m] |
| q | Nutzlänge von (70), Ausleuchtlänge [m] |
| v | Hubgeschwindigkeit [m/s] |
| | |
| D | erforderliche Strahlungsdosis [J/m$^2$] |
| P | Strahlungsleistung der Lichtquellen [W] |

**Patentansprüche**

1.  Monitorsystem (10) mit einem, einen Einführschacht (12) aufweisenden Gehäuse (11) und mit einer, einen Monitor (53) aufweisenden Anzeigevorrichtung (51), wobei die Anzeigevorrichtung (51) zwischen einer Betriebsposition (52), in der sie vollständig aus dem Einführschacht (12) des Gehäuses (11) ausgefahren ist und einer Lagerposition, in der sie vollständig im Einführschacht (12) innerhalb des Gehäuses (11) angeordnet ist, in einer Einfahrhubrichtung (55) und zurück mittels einer Hebevorrichtung (61) verfahrbar ist, wobei,

    - im Einführschacht (12) mindestens eine optische Strahlungsvorrichtung (70) angeordnet ist, deren Wellenlänge der optischen Strahlung im Bereich von 240 Nanometer bis 280 Nanometer einschließlich liegt,
- in einer Längsrichtung (5) des Monitorsystems (10) die Nutzlänge der optischen Strahlungsvorrichtung (70) mindestens der Länge der Anzeigevorrichtung (51) entspricht,
- zwischen der optischen Strahlungsvorrichtung (70) und einer Einfahröffnung (21) des Einführschachts (12) eine flexible, an die Anzeigevorrichtung (51) anlegbare Sichtschutzblende (82) angeordnet ist, deren Länge mindestens der Länge der Anzeigevorrichtung (51) entspricht und
- im Gehäuse (11) ein Gehäuseschutzschalter angeordnet ist, der die optische Strahlungsvorrichtung (70) beim Öffnen des Gehäuses (11) abschaltet.

2. Monitorsystem (10) nach Anspruch 1, **dadurch gekennzeichnet, dass** der Einführschacht (12) zumindest bei Lage der Anzeigevorrichtung (51) in der Lagerposition mittels einer Verschlussklappe (31) verschließbar ist.

3. Monitorsystem (10) nach Anspruch 2, **dadurch gekennzeichnet, dass** in der Lagerposition die Anzeigevorrichtung (51) von der geschlossenen Verschlussklappe (31) weiter entfernt ist als die Sichtschutzblende (82).

4. Monitorsystem (10) nach Anspruch 1, **dadurch gekennzeichnet, dass** die Anzeigevorrichtung (51) im Einführschacht (12) in vertikaler Richtung verfahrbar ist.

5. Monitorsystem (10) nach Anspruch 1, **dadurch gekennzeichnet, dass** es ein Sensorsystem (40) mit einem hubabhängigen Schaltabstand (44) zum bistabilen Schalten der optischen Strahlungsvorrichtung (70) aufweist, wobei der Schaltabstand (44) mindestens der Höhe der Anzeigevorrichtung (51) in der Einfahrrichtung (55), reduziert um das Produkt aus dem doppelten Abstandes einer Lichtquelle (75) der optischen Strahlungsquelle (70) von einer Anzeigeebene (59) und dem Tangens des halben Öffnungswinkels der optischen Strahlungsquelle (70) beträgt.

6. Monitorsystem (10) nach Anspruch 5, **dadurch gekennzeichnet, dass** die optische Strahlungsquelle (70) beim Betrieb der Hebevorrichtung (61) mittels eines ersten Signals des Sensorsystems (40) einschaltbar ist und nach dem Fördern der Anzeigevorrichtung (51) mittels eines zweiten, von einem vorgegebenen Schaltabstand (44) abhängigen Signals des Sensorsystems (40) abschaltbar ist.

7. Monitorsystem (10) nach Anspruch 1, **dadurch gekennzeichnet, dass** in der Einfahrhubrichtung (55) der Abstand der Sichtschutzblende (82) zu einer Normalen zur Anzeigeebene (59) der Anzeigevorrichtung (51) durch eine Lichtquelle (75) der optischen Strahlungsvorrichtung (70) größer ist als das Produkt des Abstandes der Lichtquelle (75) zur Anzeigeebene (59), multipliziert mit dem Tangens der Differenz des halben Öffnungswinkels ($\alpha$) der optischen Strahlungsvorrichtung (70) und eines Deviationswinkels ($\delta$) der optischen Strahlungsvorrichtung (70), wobei der Deviationswinkel ($\delta$) von der Normalen zur Anzeigeebene (59) durch die Lichtquelle (75) und die Winkelhalbierende des Abstrahlwinkels ($\alpha$) der Lichtquelle (75) begrenzt wird.

8. Monitorsystem (10) nach Anspruch 1, **dadurch gekennzeichnet, dass** die Verfahrgeschwindigkeit der Anzeigevorrichtung (51) kleiner ist als die Strahlungsleistung der optischen Strahlungsvorrichtung (70), dividiert durch das Produkt aus der minimal erforderlichen Strahlungsdosis und der quer zur Einfahrhubrichtung (55) orientierten Länge der in der Anzeigeebene (59) der Anzeigevorrichtung (51) mittels der optischen Strahlungsvorrichtung (70) ausgeleuchteten Fläche.

## Claims

1. Monitor system (10) with a housing (11) comprising an insertion shaft (12) and with a display device (51) comprising a monitor (53), wherein the display device (51) is movable between an operating position (52), in which it is fully extended out of the insertion shaft (12) of the housing (11), and a storage position, in which it is fully arranged in the insertion shaft (12) within the housing (11), in a retraction stroke direction (55) and back by means of a lifting device (61), wherein

- at least one optical radiation device (70) whose wavelength of the optical radiation lies in the range from 240 nanometres up to and including 280 nanometres is arranged in the insertion shaft (12),
- in a longitudinal direction (5) of the monitor system (10), the effective length of the optical radiation device (70) corresponds at least to the length of the display device (51),
- a flexible optical protection screen (82) that can be pressed against the display device (51), the length of which corresponds at least to the length of the display device (51), is arranged between the optical radiation device (70) and a retraction opening (21) of the insertion shaft (12), and
- a housing circuit breaker is arranged in the housing (11), which switches off the optical radiation device (70) when the housing (11) is opened.

2. The monitor system (10) according to claim 1, **characterized in that** the insertion shaft (12) is closable by means of a cover (31) at least when the display device (51) is in the storage position.

3. The monitor system (10) according to claim 2, **characterized in that**, in the storage position, the display device (51) is further away from the closed cover (31) than the optical protection screen (82).

4. The monitor system (10) according to claim 1, **characterized in that** the display device (51) is movable in a vertical direction in the insertion shaft (12).

5. The monitor system (10) according to claim 1, **characterized in that** it comprises a sensor system (40) with a stroke-dependent switching distance (44) for a bistable switching of the optical radiation device (70), wherein the switching distance (44) is at least the height of the display device (51) in the retraction direction (55), reduced by the product of twice the distance of a light source (75) of the optical radiation source (70) from a display plane (59) and the tangent of half the aperture angle of the optical radiation source (70).

6. The monitor system (10) according to claim 5, **characterized in that** the optical radiation source (70) can be switched on during operation of the lifting device (61) by means of a first signal of the sensor system (40) and can be switched off after the conveyance of the display device (51) by means of a second signal of the sensor system (40) which depends on a predetermined switching distance (44).

7. The monitor system (10) according to claim 1, **characterized in that**, in the retraction stroke direction (55), the distance of the optical protection screen (82) from a normal to the display plane (59) of the display device (51) through a light source (75) of the optical radiation device (70) is greater than the product of the distance of the light source (75) from the display plane (59), multiplied by the tangent of the difference between half the aperture angle ($\alpha$) of the optical radiation device (70) and a deviation angle ($\delta$) of the optical radiation device (70), wherein the deviation angle ($\delta$) from the normal to the display plane (59) is limited by the light source (75) and the angle bisector of the radiation angle ($\alpha$) of the light source (75) .

8. The monitor system (10) according to claim 1, **characterized in that** the speed of movement of the display device (51) is less than the radiant power of the optical radiation device (70) divided by the product of the minimum required radiation dose and the length, oriented transversely to the retraction stroke direction (55), of the area illuminated in the display plane (59) of the display device (51) by means of the optical radiation device (70).

**Revendications**

1. Système de moniteur (10) avec un boîtier (11) comportant un compartiment d'introduction (12) et avec un dispositif d'affichage (51) comportant un écran (53), sachant que le dispositif d'affichage (51) peut être déplacé dans une direction de course d'entrée (55) et retour au moyen d'un dispositif de levage (61), entre une position opérationnelle (52), dans laquelle il est complètement sorti du compartiment d'introduction (12) du boîtier (11) et une position de support, dans laquelle il est disposé complètement dans le compartiment d'introduction (12) à l'intérieur du boîtier (11), sachant que

- dans le compartiment d'introduction (12) est disposé au moins un dispositif de rayonnement optique (70), dont la longueur d'ondes du rayonnement optique se situe dans la plage de 240 nanomètres à 280 nanomètres inclus,
- dans une direction longitudinale (5) du système de moniteur (10), la longueur utile du dispositif de rayonnement optique (70) correspond au moins à la longueur du dispositif d'affichage (51),
- entre le dispositif de rayonnement optique (70) et une ouverture d'entrée (21) du compartiment d'introduction (12), est disposé un écran de protection visuelle (82) souple, pouvant s'appliquer au dispositif d'affichage (51), dont la longueur correspond au moins à la longueur du dispositif d'affichage (51), et
- un disjoncteur de protection de boîtier est disposé dans le boîtier (11), qui déconnecte le dispositif de rayonnement optique (70) lors de l'ouverture du boîtier (11).

2. Système de moniteur (10) selon la revendication 1, **caractérisé en ce que** le compartiment d'introduction (12) peut être fermé au moyen d'un couvercle de fermeture (31) au moins lors du positionnement du dispositif d'affichage (51) dans la position de support.

3. Système de moniteur (10) selon la revendication 2, **caractérisé en ce que** dans la position de support, le dispositif d'affichage (51) est plus éloigné du couvercle de fermeture fermé (31) que l'écran de protection visuelle (82).

4. Système de moniteur (10) selon la revendication 1, **caractérisé en ce que** le dispositif d'affichage (51) peut être déplacé dans la direction verticale dans le compartiment d'introduction (12).

5. Système de moniteur (10) selon la revendication 1, **caractérisé en ce qu'**il comporte un système de détection (40) avec une distance de commutation (44) en fonction de la course pour la connexion bistable du dispositif de rayonnement optique (70), sachant que la distance de commutation (44) est au moins de la hauteur du dispositif d'affichage (51) dans la direction d'entrée (55), réduite du produit obtenu à partir de la distance double d'une source lumineuse (75) de la source de rayonnement optique (70) d'un plan d'affichage (59) et de la tangente du demi angle d'ouverture de la source de rayonnement optique (70).

6. Système de moniteur (10) selon la revendication 5, **caractérisé en ce que** la source de rayonnement optique (70) peut être connectée lors du fonctionnement du dispositif de levage (61) au moyen d'un premier signal du système de détection (40) et déconnectée après le déplacement du dispositif d'affichage (51) au moyen d'un deuxième signal prédéfini du système de détection (40) en fonction de la distance de commutation (44).

7. Système de moniteur (10) selon la revendication 1, **caractérisé en ce que** dans la direction de la course d'entrée (55), la distance de l'écran de protection visuelle (82) à une normale par rapport au plan d'affichage (59) du dispositif d'affichage (51) à travers une source lumineuse (75) du dispositif de rayonnement optique (70) est plus grande que le produit de la distance de la source lumineuse (75) par rapport au plan d'affichage (59), multipliée par la tangente de la différence du demi angle d'ouverture ($\alpha$) du dispositif de rayonnement optique (70) et d'un angle de déviation ($\delta$) du dispositif de rayonnement optique (70), sachant que l'angle de déviation ($\delta$) du de la normale par rapport au plan d'affichage (59) est limité par la source lumineuse (75) et la bissectrice de l'angle de rayonnement ($\alpha$) de la source lumineuse (75).

8. Système de moniteur (10) selon la revendication 1, **caractérisé en ce que** la vitesse de déplacement du dispositif d'affichage (51) est plus petite que la puissance de rayonnement du dispositif de rayonnement optique (70), divisée par le produit obtenu à partir de la dose de rayonnement au minimum nécessaire et de la longueur, orientée transversalement à la direction de la course d'entrée (55) de la surface éclairée dans le plan d'affichage (59) du dispositif d'affichage (51) au moyen du dispositif de rayonnement optique (70).

**Fig. 1**

**Fig. 2**

**Fig. 3**

EP 4 170 221 B1

**Fig. 4**

72   70   71   74   73

q   p

**Fig. 5**

81
82
δ
α

c
70

59   a   75

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3604889 A1
- CN 109171201 A